# EUROPÄISCHE PATENTSCHRIFT

(11) **EP 0 594 570 B1**
(45) Veröffentlichungstag und Bekanntmachung des Hinweises auf die Patenterteilung: **26.07.1995**
(21) Anmeldenummer: 90913811.7
(22) Anmeldetag: 07.09.1990
(51) Int. Cl.: A61K 31/785, A61K 31/74, A61K 9/16, A61K 9/20

(54) **COLESTYRAMIN ALS LIPIDSENKER ENTHALTENDE PRÄPARATE**
PREPARATIONS CONTAINING COLESTYRAMINE FOR REDUCING LIPID LEVELS
MEDICAMENTS RENFERMANT DE LA COLESTYRAMINE POUR REDUIRE LES NIVEAUX DE LIPIDES

(30) Priorität: 09.09.1989 DE 3930168; 09.09.1989 DE 3930206
(43) Veröffentlichungstag der Anmeldung: 04.05.1994
(73) Patentinhaber: KNOLL AG, D-67061 Ludwigshafen (DE)
(72) Erfinder: MOEST, Thomas, D-2082 Moorrege (DE); SPIEGEL, Erwin, D-6945 Hirschberg (DE)
(74) Vertreter: Karau, Wolfgang, Dr.
(86) Internationale Anmeldenummer: EP9001514
(87) Internationale Veröffentlichungsnummer: WO9103249

(56) Entgegenhaltungen:
- FR-A- 2 110 458
- FR-A- 2 382 893
- US-A- 4 814 354

## Beschreibung

Die vorliegende Erfindung betrifft Colestyramin als Lipidsenker in hoher Konzentration enthaltende Präparate in Form von Mikrotabletten, deren längster Teilchendurchmesser 1 bis 4 mm beträgt.

Colestyramin, ein in der Medizin u.a. als Lipidsenker bekanntes Mittel, ist ein Anionen-Austauscherharz aus einem Copolymerisat aus Styrol und Divinylbenzol, das quaternäre Ammoniumgruppen enthält.

Es wird bisher nur als Pulver in den Handel gebracht (s. Rote Liste 1990, Verzeichnis von Fertigarzneimitteln der Mitglieder des Bundesverbandes der Pharmazeutischen Industrie e.V.). Ein Nachteil dieser Darreichungsform ist, daß das Colestyramin bei der Einnahme einen unangenehmen sandigen Geschmack im Mund hinterläßt (siehe beispielsweise Knodel et al, Medical Toxicology 2 (1987) 10, S. 13, 1. Absatz von Kap. 1.2, in dem nachteilige Wirkungen von Lipidsenkern behandelt werden). Da das Colestyramin heute üblicherweise in Einzeldosen von etwa 4 g zwei- bis achtmal pro Tag eingenommen werden muß, führt dies häufig dazu, daß die Patienten weniger als die vorgeschriebene Dosis einnehmen oder sogar die Therapie mit Colestyramin abbrechen (s. EP-A 261 693, S. 2, Zeile 7-8).

An Versuchen, Colestyramin in anderer Darreichungsform anzubieten, hat es nicht gefehlt. So werden in der US-A 4,814,354 Colestyramin enthaltende Süßwaren, in der EP-A 347 014 ein Gebäck mit Colestyramin und in der DE-A 38 08 191 wäßrige Colestyramin enthaltende Suspensionen beschrieben. Der unangenehme sandige Geschmack läßt sich dadurch aber nicht beseitigen. FR 2 382 893 beschreibt schwach colestyraminhaltige Präparate zum Magenschutz. Für die zur Lipidsenkung benötigten Colestyraminmengen sind sie nicht geeignet.

Der Erfindung lag die Aufgabe zugrunde, Colestyramin als Lipidsenker enthaltende Präparate in einer Darreichungsform bereit zustellen, die die oben genannten Nachteile nicht aufweisen.

Demgemäß wurden 80 bis 99 % Colestyramin als Lipidsenker enthaltende Mikrotabletten gefunden, deren längster Teilchendurchmesser 1 bis 4 mm beträgt.

Colestyramin läßt sich zu Mikrotabletten verpressen. Die Mikrotablette ist in der Regel zylindrisch und hat eine Größe von 1 bis 4 mm (sowohl Höhe wie auch Durchmesser), insbesondere von 2,0 bis 3,5 mm. Daneben sind prinzipiell auch andere Formen wie Kugeln möglich.

Die Formen kann man in üblicher, beispielsweise in der in der EP-A 166 315 beschriebenen Weise herstellen. Bei der Formulierung kann man die gebräuchlichen galenischen Hilfsmittel wie Bindemittel, Hilfsstoffe, Konservierungsmittel, Netzmittel, Fließregulierungsmittel, Schmiermittel und/oder Antioxidantien (s. z.B. H. Sucker et al: Pharmazeutische Technologie", Thieme Verlag Stuttgart, (1978)) zusetzen. Weiterhin kann man die Formen mit den üblichen galenischen Überzügen versehen.

Als bevorzugtes Bindemittel beim Verpressen verwendet man mikrokristalline Cellulose, das von 2 bis 20, vorzugsweise von 3 bis 8 Gew.-% im Arzneimittel enthalten ist. Beim Granulieren setzt man vorteilhaft Cellulosederivate wie Methylcellulose, Hydroxypropylmethylcellulose, Hydroxyethylcellulose und Polyvinylpyrrolidon in einer Menge von 2 bis 10, vorzugsweise 3 bis 6 Gew.-%, ein.

Die Dosierung hängt vom Alter, Zustand und Gewicht des Patienten ab. In der Regel beträgt die tägliche Wirkstoffdosis zwischen 0,03 und 0,4 g/kg Körpergewicht.

### Beispiele

### Beispiel 1

13,5 kg Colestyramin (Fa. Röhm & Haas Deutschland GmbH, Colestyramin 40 ») wurden in einem pharmaüblichen Hochleistungsmischer mit 675 g direkttablettierbarer Lactose und 600 g mikrokristalliner Cellulose vermischt. Dann wurden 75 g hochdisperses Siliciumdioxid und 150 g Magnesiumstearat zugegeben und weitergemischt. Diese Preßmischung wurde dann zu Mikrotabletten mit 3,5 mm Durchmesser und gleicher Höhe bei einer Einzelmasse von 30 mg verpreßt.

### Beispiel 2

13,5 kg Colestyramin (s.o.) wurden in einem pharmaüblichen Hochleistungsmischer mit Zerhacker mit einer Lösung von 0,7 kg Polyvinylpyrrolidon (mittlere Molekularmasse 25.000) in 2,1 kg Isopropanol vensetzt und granuliert. Nach dem Trocknen bei 50°C wurde über ein oszillierendes Sieb mit 0,8 mm Maschenweite gesiebt. Das Granulat wurde dann mit 70 g hochdispersem Siliciumdioxid und 70 g Magnesiumstearat vermischt. Die preßfertige Masse wurde zu Mikrotabletten mit 3 mm Durchmesser und gleicher Höhe bei einer Einzelmasse von 17 mg verpreßt.

## Patentansprüche

1. Colestyramin enthaltendes Präparat, dadurch gekennzeichnet, daß es in Form von Mikrotabletten vorliegt, deren längster Teilchendurchmesser 1 bis 4 mm beträgt, und 80 bis 99 % Colestyramin sowie gebräuchliche galenische Hilfsstoffe enthält.

## Claims

1. A colestyramine-containing product, which is in the form of microtablets whose longest particle diameter is from 1 to 4 mm and contains from 80 to 99% colestyramine and conventional pharmaceutical auxiliaries.

## Revendications

1. Préparation contenant de la colestyramine caractérisée par le fait qu'elle se présente sous forme de microtablettes dont le diamètre de particules le plus grand est de 1 à 4 mm et contient 80 à 99 % de colestyramine ainsi que des auxiliaires galéniques usuels.
